# EUROPÄISCHE PATENTANMELDUNG

(11) **EP 0 870 472 A1**
(43) Veröffentlichungstag der Anmeldung: **14.10.1998**
(21) Anmeldenummer: 97810200.2
(22) Anmeldetag: 07.04.1997
(51) Int. Cl.: A61B 17/32

(54) **Diamant-Skalpell zum Öffnen der Hirnhaut**

(71) Anmelder: Anton Meyer & Co.AG, 2560 Nidau (CH)
(72) Erfinder: Meyer, Hans-Jörg, 2563 Ipsach (CH); Ziemer, Frank, 2562 Port (CH)
(74) Vertreter: Münch, Otto

(57) **Zusammenfassung**

Das Skalpell hat einen Griff (2), in welchem eine Fassung (6) mit einer Diamant-Klinge (7) verschiebbar gelagert ist. Am Griff (2) ist ein Fusshalter (4) mit geschlitztem Fuss (5) befestigt. Die Fassung (6) kann durch einen Tambour (3) in zwei Stellungen so verstellt werden, dass die Spitze (8) der Klinge (7) entweder 0,3 mm über die Unterseite des Fusses (5) vorsteht oder sich innerhalb der Kontur des Fusses (5) befindet. Mit dem Skalpell kann die Hirnhaut einfach und sicher geöffnet werden.

## Beschreibung

Das Öffnen der Hirnhaut ist ein Verfahren, das seit den Anfängen der Neurochirurgie besteht. Seit Ende des 19. Jahrhunderts, aber vor allem nach dem Anbruch der modernen Neurochirurgie wurden vor allem Scheren bei der opertaiven Öffnung der Hirnhaut eingesetzt. Desweiteren kommen auch feine chirurgische Zangen zum Einsatz, um die unversehrte Hirnhaut von der arachnoido-pialen Fläche wegzuziehen. Die Öffnung wird durch ein Messer vorgenommen. Danach wird die Hirnhaut mit Rundspitzenscheren eingeschnitten. Oftmals ist es von Vorteil, eine kleine feuchte Watte durch die erste Öffnung unter die Hirnhaut zu legen, um somit die darunter liegende Hirnrinde zu schützen. Es ist auch möglich, eine kleine gerillte Sonde und ein Messer zur Öffnung der Hirnhaut zu benutzen. Beim wiederholten Öffnen sollte das Messer wegen der Synchien zwischen pachi- und leptomeningealen Flächen oder zwischen der Hirnhautrinde und den -gefässen vorsichtig eingesetzt werden.

Das Öffnen der Hirnhaut kann ein leichtes und sicheres, aber auch ein sehr schwieriges Verfahren sein. Die Schwierigkeiten hängen von verschiedenen Faktoren ab:
- Hirnhautdicke
- Zusätzliche Hirnhautsinusen
- Brückenadern innerhalb der Hirnhautfläche
- Aggressivität des geschwollenen und deshalb schwachen Gehirns und dem virtuellen subarchanoidalen Raum. In einem solchen Zustand wird die Öffnung der Hirnhaut zu einem schwierigen und potentiell gefährlichen Verfahren.

Der vorliegenden Erfindung liegt die Aufgabe zugrunde, ein Instrument anzugeben, mit welchem das Öffnen der Hirnhaut einfacher und sicherer ist. Diese Aufgabe wird durch die Merkmalskombination des Anspruchs 1 gelöst.

Nachfolgend wird ein Ausführungsbeispiel der Erfindung anhand der Zeichnungen erläutert. Darin zeigt:
Figur 1 eine Ansicht eines erfindungsgemässen Skalpells,
Figuren 2 und 3 Ansichten der Klinge,
Figuren 4 und 5 Ansichten des Fusshalters, und
Figuren 6 und 7 das Skalpell beim Öffnen der Hirnhaut.

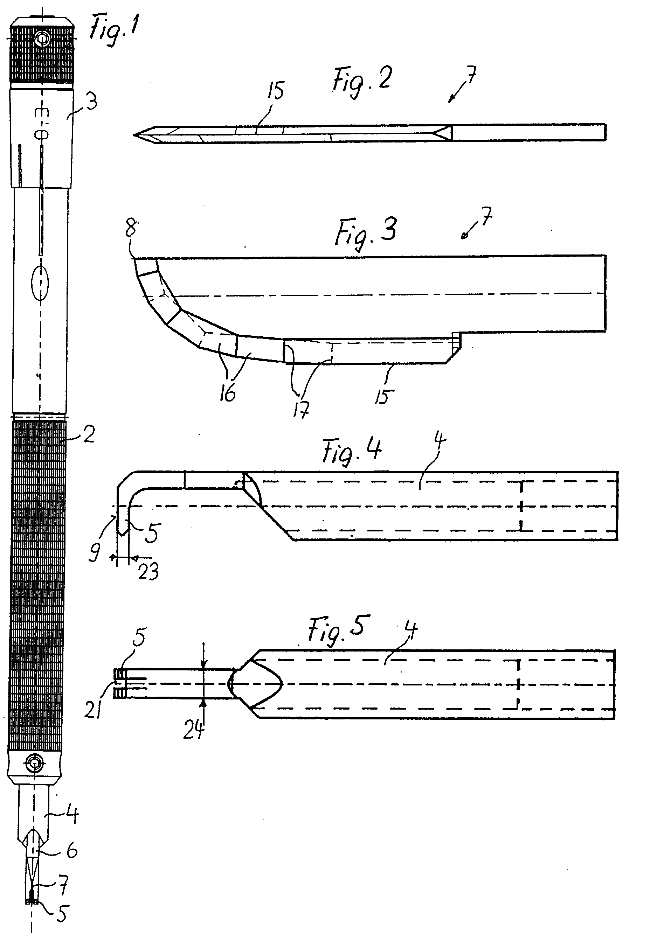

Das Skalpell gemäss Figur 1 hat einen Griff 2 mit einem Schalttambour 3. Am Griff 2 ist ein Fusshalter 4 mit einem geschlitzten Fuss 5 befestigt. Im Griff 2 ist eine stabförmige Fassung 6 längsverschiebbar und gegen Verdrehen gesichert. In der Fassung 6 ist eine Diamantklinge 7 befestigt. Die Fassung 6 ist mittels des Schalttambours 3 in zwei achsiale Stellungen schaltbar, zum Beispiel in der in der CH-PS-671 872 beschriebenen Art. Diese Schrift wird zum integrierenden Bestandteil dieser Anmeldung erklärt, sodass der Schaltmechanismus hier nicht erläutert zu werden braucht. In der ersten Stellung überragt die Spitze 8 der Diamantklinge 7 die Unterseite 9 des Fusses 5 um 0,3 mm. In der zweiten Stellung ist die Spitze 8 innerhalb der Kontur des Fusses 5.

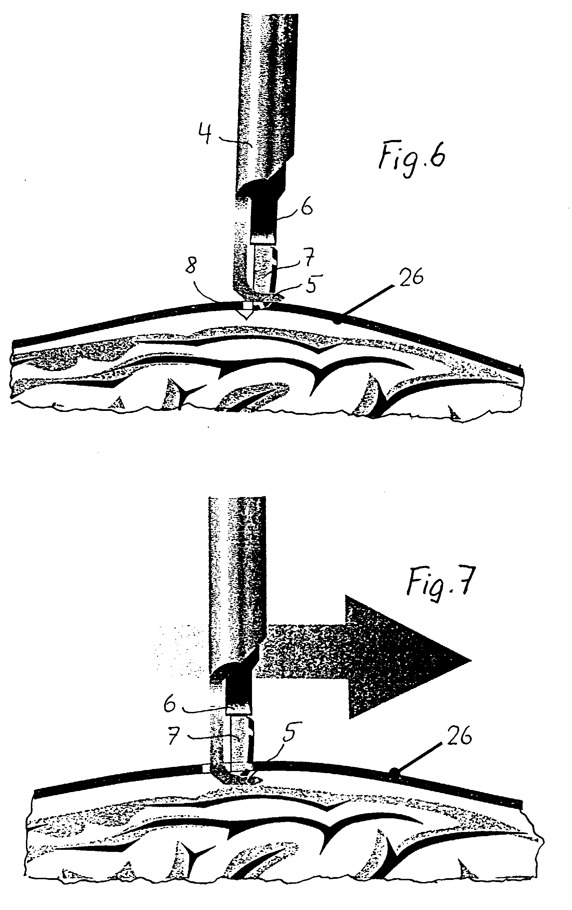

In Figuren 2 und 3 ist die Klinge 7 vergrössert dargestellt. Sie ist 0,2 mm dick und hat eine bogenförmige Schnittkante 15, welche gebildet wird durch aneinandergereihte, ebene Facetten 16. Die Kanten 17 zwischen aneinandergrenzenden Facetten 16 sind auf den beiden Seiten der Klinge 7 gegeneinander versetzt. Der Fusshalter 4 mit dem Fuss 5 ist in Figuren 4 und 5 vergrössert dargestellt. Die Klinge 7 ragt durch den Schlitz 21 des Fusses 5. Das freie Ende des Fusses 5 ist von der Unterseite 9 und der Oberseite 22 unter 45° abgeschrägt. Die Dicke 23 des Fusses 5 beträgt 0,6 mm und die Breite 24 1,5 mm.

Das Skalpell hat zusätzlich eine nicht dargestellte Schutzhülse mit einer abgestuften Durchgangsbohrung, welche auf das vordere Ende des Griffs 2 aufsteckbar ist, den Fusshalter 4 mit Spiel umgibt und den Fuss 5 überragt.

In Figur 6 ist das vordere Ende des Skalpells beim Aufschneiden der Hirnhaut 26 dargestellt. Die Klinge 7 ist in der ersten Stellung. Die Hirnhaut wird durch eine kleine chirurgische Zange weggezogen und mit der Klinge einige Millimeter weit eingeschnitten. Hierauf wird die Klinge 7 in die zweite Stellung zurückgezogen und der Fuss 5 durch den Initialschnitt unter die Hirnhaut 26 eingeschoben. Mit dem Fuss 5 wird nun die Hirnhaut 26 nach aussen gezogen und die Klinge 7 in der gewünschten Richtung gezogen. Sie schneidet leicht und genau die Pachimeningis ein (Figur 7). Das beschriebene Skalpell ist in folgenden Fällen sehr nützlich:
- Öffnung der Spinalhirnhaut
- "Key-Hole"-Chirurgie
- Chirurgie in Posterior Fossa
- Chirurgie in sitzender oder halbsitzender Position

Das beschriebene Skalpell und das beschriebene Vorgehen hat gegenüber dem traditionellen Verfahren mehrere Vorteile:
- Die Hirnhaut kann mit dem Fuss 5 zurückgezogen werden.
- Die arachoide Hirnrinde wird geschützt, weil die Klingenspitze 8 beim Schnitt innerhalb der Kontur des Fusses 5 ist.
- Das Diamantskalpell erlaubt einen glatten, feinen und präzisen Schnitt mit einer ewig scharfen Klinge (bei sachgemässer Behandlung).
- Das Skalpell gewährleistet durch den Fuss eine Fortgangsblockierung vor Hindernissen. Sein mikroskopisch kleiner Fuss geht weiter, bis er ein Hindernis erreicht (wie beispielsweise die laterale Ecke des SSS). Eine zufällige Beschädigung (Verletzung) wird somit unwahrscheinlich. Sollte dies gleichwohl einmal passieren, wird der Riss so klein sein, dass er mit einem einzelnen vaskulären Stich (7-0) leicht verschlossen werden kann.
- Durch die bogenförmige Schnittkante wird eine gleichmässigere Schnittführung gewährleistet. Auch bei leichten Kippbewegungen ist ein sauberer Schnitt möglich.

## Patentansprüche

1. Diamantskalpell zum Öffnen der Hirnhaut (26), umfassend einen Griff (2), eine in einer Fassung (6) gefasste Klinge (7) sowie einen am Griff (2) befestigten, sich beidseitig der Klinge (7) erstreckenden, geschlitzten Fuss (5), wobei die Klinge (7) in mindestens zwei Stellungen verschiebbar ist, wobei die Klingenspitze (8) in einer ersten Stellung die Unterseite (9) des Fusses (5) überragt und sie in einer zweiten Stellung innerhalb der Kontur des Fusses (5) ist.

2. Skalpell nach Anspruch 1, wobei die Schnittkante (15) der Klinge (7) bogenförmig geschliffen ist.

3. Skalpell nach Anspruch 2, wobei der bogenförmige Schliff durch ebene, aneinandergrenzende Facetten (16) gebildet ist.

4. Skalpell nach Anspruch 3, wobei die Kanten (17) der Facetten (16) auf den beiden Seiten der Klinge (7) gegeneinander versetzt sind.

5. Skalpell nach einem der Ansprüche 1 bis 4, wobei die Dicke (23) des Fusses (5) etwa 0,6 mm beträgt.

6. Skalpell nach einem der Ansprüche 1 bis 5, wobei der vordere freie Rand des Fusses (5) sowohl von der Unterseite (9) als auch von der Oberseite (22) her abgeschrägt ist.

7. Skalpell nach einem der Ansprüche 1 bis 6, wobei die Klingenspitze (8) in der ersten Stellung die Unterseite (9) des Fusses (5) um höchstens 0,5 mm, vorzugsweise um etwa 0,3 mm überragt.

8. Skalpell nach einem der Ansprüche 1 bis 7, wobei es eine zusätzlich auf das vordere Ende des Griffs (2) aufsteckbare Schutzhülse aufweist, welche im aufgesteckten Zustand den Fuss (5) überragt.
